# EUROPEAN PATENT APPLICATION

(11) **EP 4 389 015 A1**
(43) Date of publication of application: **26.06.2024**
(21) Application number: 22215902.2
(22) Date of filing: 22.12.2022
(51) Int. Cl.: A61B 8/06, A61B 8/08, A61B 8/00

(54) **ULTRASOUND SYSTEM**

(71) Applicant: Pulsify Medical, 3001 Leuven (BE); Imec VZW, 3001 Leuven (BE)
(72) Inventor: STOFFELS, Steve, 3001 Leuven (BE); CHEYNS, David, Heffen (BE); ROCHUS, Veronique, Embourg (BE); ROTTENBERG, Xavier, Kessel-Lo (BE); MYNY, Kris, Heusden-Zolder (BE)
(74) Representative: DTS Patent- und Rechtsanwälte Schnekenbühl und Partner mbB

(57) **Abstract**

The invention relates to an ultrasound system (1) for intercostal imaging of organs, in particular for cardiac monitoring, comprising a transducer array (3) with a plurality of transducer array elements (3a), a control unit (40), which is coupled to the transducer array (3), for controlling the transducer array (3), and a data processing unit (41) for receiving data from the transducer array (3) and processing the received data. Therein, the ultrasound system (1) further comprises a complexity reduction module (42), which is configured to define a plurality of sub-arrays (4) of transducer array elements (3a) and to control individual sub-arrays (4) as functional units for sending ultrasound pulses and/or receiving ultrasound signals. The invention also relates to a method for operating an ultrasound system (1), wherein a plurality of sub-arrays (4) is defined within a transducer array (3), and the individual sub-arrays (4) are controlled as functional units for sending ultrasound pulses and/or receiving ultrasound signals.

## Description

The present invention relates to an ultrasound system, in particular for cardiac monitoring.

Wearable ultrasound monitor systems monitor cardiac function over time. The ultrasound monitor can be body worn in a patch type format.

US 2016/0136462 A1 discloses a wearable ultrasound device and method of using the device including a power controller with a power source and at least one integrated circuit that delivers electrical power to an applicator. The applicator is electrically coupled to the power controller and a surface of the applicator transmits ultrasound to a wearer for a given duration. The applicator includes radio frequency (RF) drive electronics, an ultrasound transducer coupled to the drive electronics, a monitoring apparatus that includes a thermal cutoff coupled to the drive electronics.

WO 2018/102828 A1 discloses an ultrasound coupling patch for use with ultrasound transducers, and more particularly to ultrasound coupling patches having a gel capture feature.

WO 2019/162485 A1 discloses a method and an apparatus for performing ultrasound measurements of a propagation medium containing non uniformities, with a transmitter/transducer with a plurality of transmitting elements for emitting ultrasound excitation pressure waves.

Furthermore, EP 3 708 263 A1 discloses a flexible ultrasound transducer for an ultrasound monitoring system for examining a curved object. The ultrasound transducer comprises an integrated circuit structure and a multi-layered structure, said multi-layered structure comprising an array of ultrasound transducing elements arranged in a first layer structure and configured for generating ultrasonic energy propagating along a main transducer axis Z and an array of control circuits arranged in a second layer structure, and wherein the array of control circuits and the integrated circuit structure are configured for operating the array of ultrasound transducing elements in said first layer structure, Further, the multi-layered structure comprises at least one flexible layer arranged so that the bending flexibility of the multi-layered structure permits the ultrasound transducer to form a continuous contact with said curved object during operation.

In ultrasound systems with a large number of ultrasound transducer elements, e.g., when a transducer array with 10.000 or 100.000 elements is used, individually sampling every single transducer element leads to massive amounts of data. The system needs accordingly high capabilities for the throughput and the processing of this data. A high amount of power is required to drive and read such an array and all its individual transducer elements.

To reduce the overall data-rate and the power consumption while maintaining sufficient image quality for the respective application, the system complexity needs to be taken into account.

It is the problem of the invention to provide an ultrasound system that allows efficient data acquisition, treatment and processing, without impacting the overall data quality negatively.

This problem is solved by an ultrasound system for intercostal imaging of organs according to claim 1 of the attached set of claims. Further advantageous embodiments are given in the dependent claims.

The ultrasound system for intercostal imaging of organs, in particular for cardiac monitoring, comprises a transducer array with a plurality of transducer array elements, a control unit, which is coupled to the transducer array, for controlling the transducer array, and a data processing unit for receiving data from the transducer array and processing the received data. The system further comprises a complexity reduction module, which is configured to define at least one or a plurality of sub-arrays of transducer array elements and to control the at least one or the plurality sub-arrays as functional units for sending at least one (or more) ultrasound pulse(s) and/or receiving at least one or more ultrasound signal(s), wherein further the complexity reduction module is configured to combine several transducer array elements into a functional unit, which then can be handled as if it were one single transducer array element, and wherein the complexity reduction module is configured to read and/or drive and/or control such functional units.

In particular, the ultrasound system can be in general an ultrasound system as disclosed by WO 2019/162485 A1. The further disclosed features can be added for example to the system as disclosed in WO 2019/162485 A1.

Thus, the system advantageously requires less computational capacities and the power consumption of the ultrasound system is reduced.

The invention is based on the idea that complexity and data throughput are reduced by a hierarchical aggregation of transducer elements. On the other hand, the ultrasound device's operation is not be inhibited or only minimally impacted.

In other words, the basic concept definition for an array with the number N rows and the number M columns (if N=M, then the array is a squared array, otherwise a rectangular array) is such, that there is a controller attached to a matrix of switches, with the switch matrix consisting of a plurality of switches, such as e.g. transistor or TFT elements (the switch elements will open or close the signal path) and this controller defines, which rows are open or have to be open. Further, the controller defines or is aware of the columns and row, which are active for signal transmission and/or receiving, and the intersection of active N and active M is defining a sub-array. This way, the addressing of the transducer array elements can be done in a less complex way, as they will be addressed in the groups of transducer array elements which are called the sub-array.

In particular and according to the invention, the complexity reduction module is configured to combine several transducer array elements into a functional unit, which then can be handled as if it were one single transducer array element, and wherein the complexity reduction module is configured to read and/or drive and/or control such functional units. This way, less transducer array elements must be controlled and/or driven at the same time and so the complexity in handling of the plurality of the transducer array elements is significantly reduced.

The avoidance of an overlap can also be done and ensured by only allowing one sub-array being active at one point of time.

The system may be configured for cardiac monitoring, which may for example comprise monitoring of cardiac activity and/or cardiac properties of a patient. Monitoring cardiac activity may comprise monitoring of parameters such as cardiac rhythm and/or cardiac output.

The system has at least one transducer array, i.e., a device or unit comprising a plurality of transducer array elements. In particular, the transducer array may have a chip design. The transducer array elements are units, which may be used as an actuator for generating an ultrasound pulse or signal. Alternatively or additionally, the transducer array elements may also be used for receiving or detecting an ultrasound pulse or signal. Generally speaking, one transducer array element may consist of one or more transducer sub-elements. Such a sub-element can be a single transducer only, which can be a piezoelectric micromachined ultrasonic transducer (pMUT) component or a capacitive micromachined ultrasonic transducer (cMUT) component. The transducer subelements can be arranged in a configuration were some of them are used for transmitting ultrasound while others are used for sensing. Alternatively, all elements are used both for transmitting and receiving. Using multiple sub-elements can increase the overall pressure output and sensitivity of the transducer elements. The configuration of the sub-elements might be in series or parallel configuration or a combination thereof.

In an embodiment, a transducer array element may for example consist of four, nine, sixteen transducer sub-elements, which may be arranged in either a 2x2, 3x3, 4x4 grid or in another suitable fashion. Also, non square configurations can be used e.g. for 6 sub-elements in a 2x3 configuration. In further embodiments, other numbers of transducer sub-elements in one transducer array element can be provided, and the transducer sub-elements of one transducer array element may be arranged in a grid or in another arrangement.

For example, one ultrasound transducer element may comprise one or more piezoelectric transducer sub-element(s), such as piezoelectric micromachined ultrasonic transducers (pMUT).

Different techniques may be used to manufacture transducer elements and/or sub-elements. For example, cMUT units may be fabricated using a polymer-based technique, such as described by Gerardo et al. in "Fabrication and testing of polymer-based capacitive micromachined ultrasound transducers for medical imaging", (Microsystems & Nanoengineering (2018) 4:19).

In order to reduce the complexity of the system, several transducer elements may be combined into a functional unit and treated as a single "element", i.e., the combined transducer array elements are driven at that same time as if they are a single unit.

The transducer array elements in the transducer array can be connected in series or parallel, such that they can be driven optimally.

In particular, the transducer array elements may be configured such that they are individually controllable.

In an embodiment of the system, the transducer array is flexible. Thus, the system can advantageously adjust its geometry to a probe or sample surface, e.g., a patient's body. In particular, the transducer array elements may be flexibly oriented towards each other.

For example, the transducer array elements may be supported by a flexible substrate such that they are arranged on a flat or curved plane. The substrate may, e.g., comprise a polymer material.

In an embodiment of the system, the transducer array is integrated into an adhesive patch for fixing the transducer array to a patient's body. The system can then advantageously be easily used, e.g., for monitoring a patient's body functions, such as cardiac functions, especially for long-term monitoring. The patch can be an adhesive patch.

The transducer array may be integrated into the patch by providing it with an adhesive area, which is suited for attaching the patch to a patient's body surface. Also, a textile or other flexible material may be provided with an adhesive material and used to fix the transducer array to the body surface. Alternatively a mechanical strap without adhesive or a combination of strap and adhesive might be provided to fix the transducer to the body surface. In particular, the patch may be configured such that it secures the transducer array in close proximity to the body surface.

Also, the ultrasound system comprises a control unit. The control unit may comprise several components, which may be structurally integrated with each other, for example in a common casing or envelope structure, or which may be implemented as structurally separated components, which may be couple to each other.

The transducer array elements are configured to generate and/or receive ultrasound, in particular ultrasound pulses or signals, based on a control signal. To provide the respective control signals for the transducer array, the control unit may comprise a send module and a receive module, which may be implemented in a driver module for the transducer array and/or for transistors, which may control the transducer array elements.

The control unit may be configured to perform different tasks such as, but not limited to, the following examples: The control unit may be configured to perform digital signal processing and/or filtering. Also, the control unit may be configured to perform operations related to storing and/or retrieving information, in particular in a memory module, such as a random access memory (RAM) module. Also, the control unit may be configured to perform operations for driving an external interface, such as an interface to a memory module, a transceiver unit, e.g., via a USB interface or a wireless interface, and/or receiving control signals from other units. The control unit may also perform operations for controlling the transducer array, e.g., through a switch linked to a transducer array element, such a switch can be a transistor or more specifically a thin-film transistor (TFT) element, or a matrix of such switches/transistors, e.g., in a TFT matrix. The TFT matrix can be a(n) (integral) part in terms of function and/or structure of the control unit. Also, the control unit may be configured to control a switch/transistor/TFT element connected to a transducer element, to generate delays for generating and/or sensing ultrasound pulses and signals, to define generated ultrasound pulses, beamforming operations, and/or to control power management. Each of said functions may be carried out and/or implemented in an ASIC design individually or in combination. Also, these functions may be implemented as modules of the control unit.

The control unit may comprise a switch, e.g. a transistor or thin-film transistor (TFT), which can be configured to control a transducer array element. In particular, an array of switches/transistors/thin-film transistors may be provided, which is coupled to the transducer array.

A TFT array (or more generally speaking the switching module) of the system is used to control the transducer array. The TFT array or the switching module can be coupled to the transducer array and especially it may be coupled to the input ports of the transducer elements.

In particular, the switch array (e.g. TFT array) may be structurally integrated with the transducer array in one component, e.g., in a common casing or envelope structure. In particular, a monolithic chip design or structure may be used to integrate TFT array and transducer array into one system component.

The control unit may consist of or comprise one or several microprocessors. Also, the control unit may comprise an application-specific integrated circuit (ASIC) design. The control unit, in particular a microprocessor of the control unit, may be fully or partially integrated into a printed circuit board (PCB). The control unit may also comprise of control circuitry integrated on the panel for the TFT array, this control circuitry will work in conjunction with but as a separate functional unit compared to the TFT switch elements, which enable the selection of the transducer elements.

The PCB might be rigid, flexible, stretchable or a hybrid combination thereof.

In an embodiment, the system further comprises a beam former module. Herein, the beam former module is configured to apply a time delay to at least one transducer array element or a plurality of transducer array elements. This allows advantageously easy control for high-resolution measurements, beam focusing and/or beam steering.

In particular, the time delay may be applied, when the transducer array element or the plurality of transducer array elements is used for sending ultrasound pulses and/or receiving ultrasound signals.

The time delay for a transducer array element or a plurality of transducer array elements may be defined relative to controlling other transducer array elements.

Also, the beam former module and/or another unit of the system may be utilized to determine the delay. In particular, the delay is determined for each transducer array element or for a plurality of transducer array elements, to which the determined delay is to be applied.

In particular, the control unit may comprise an ASIC for implementing a beam former control, and/or a design for a signal processing algorithm, e.g., for applying spatial and/or temporal sparsity conditions for sending ultrasound pulses and/or for collecting or providing data (related to the measurements performed by the ultrasound device and the ultrasound pulses and signals). In particular, the beam former control can for example comprise an electronic delay and sum circuit that can apply different delays to a certain number of transducer array elements, for example 8 to 32, e.g. 14, 15, 16, 17 or 18 transducer array elements or functional units comprising several connected transducer array elements (e.g. 14, 15, 16, 17 or 18). It may sum the output of these channels into one single output value. In particular, by applying specific delays to each functional unit of transducer array elements, an ultrasound pulse may be generated in a coordinated fashion such that a specific profile for the ultrasound beam is obtained. Also, elements receiving ultrasound signals may be controlled by a beam former in a coordinated way to provide targeted measurements inside a sample volume.

The data processing unit may be implemented in different ways. It may for example comprise a microprocessor. In particular, it may comprise an ASIC design. Also, the data processing unit and the control unit may be implemented as software modules that are run, at least partially, on a common computational unit. For example, a processing unit may read from a memory and execute instructions for performing operations to implement the data processing unit and/or control unit.

Also, the data processing unit may comprise one or several analog-digital converters (ADC) and/or digital-analog converters (DAC). These converters are configured to link analog and digital channels to each other and allow both digital data collection and treatment, and treating analog control and detection signals. A time gain controller may be implemented using a DAC element and/or a different architecture.

The complexity reduction module may be implemented as software module, comprising instructions for computational operations that implement the function of complexity reduction. This software module may in particular be implemented as a module of the control unit. Specifically, the complexity reduction module may be comprised, at least partially, by the control unit and/or by the data processing unit.

The complexity reduction module implements functions to reduce data and sensor complexity and/or quantity. Such functions may impact the generation and/or the detection of ultrasound pulses and signals by the transducer array, or they may influence the data acquisition and/or treatment.

The coupling between the transducer array and the control unit may comprise a wire connection, in particular an interface for data and/or power transmission between the transducer array and the control unit. In particular, the control unit and/or a part of the control unit may be arranged on the sensor array or may be integrated with the sensor array into one common part. The sensor array can be embodied especially by means of the combination of a switch (e.g. TFT) array and a transducer array.

In particular, there is a wire connection and/or a wireless interface provided, if the control unit controls the sensor array. If a wireless sensor array is implemented, the control unit may be arranged on the sensor array and/or integrated into one component with the sensor array.

In particular, if the sensor array is connected to another element by a wire, at least part of the controlling of the sensor array may be implemented on the sensor array and/or integrated into one component with the sensor array. Also, an additional control unit may be provided, e.g., in a separate PC or control unit, when there is access through wires to the sensor array. A sensor array is for example a combination of a transducer array and a TFT array. Parts of the controller can be integrated on a TFT array by an integrated circuit design or by bonding ICs to the TFT.

In an embodiment, the ultrasound system further comprises a power source unit for providing energy to the transducer array and/or the control unit. The system can thus be advantageously used in a very flexible manner, in particular when a portable power source is provided. For example, the power source may comprise a battery, which is integrated in a portable device as a component of the system.

Rather than operating the transducer array as a single whole and using each element as an individual unit, a sub-array scanning architecture may be used, i.e., groups of transducer array elements are combined to functional units. In particular, neighboring elements are combined.

In particular, a control algorithm may be provided for controlling each sub-array within the transducer array. The control algorithm may control, for each sub-array, a location of the sub-array within the plurality of transducer array elements. Also, the control algorithm may control, for each sub-array, a focus depth and/or a steering angle for an ultrasound sending beam profile and/or an ultrasound receiving beam profile implemented by the sub-array of transducer array elements; such a beamforming algorithm may be implemented as a software module for a programmable unit and/or by chip design, e.g., in an ASIC.

When individual sub-arrays are controlled as functional units for sending ultrasound pulses and/or receiving ultrasound signals, the system may for example be configured to perform ultrasound based measurements of a sample by using the sub-array as a unit cell for sending and/or receiving ultrasound pulses and/or receiving ultrasound signals. In particular, ultrasound signals are detected as reflections of a previously generated ultrasound pulses, and imaging or other data processing is performed by mapping the reflected signal and its intensity to specific areas or locations.

The transducer array elements of a sub-array work as a combined "functional unit", collecting 1D depth data sets by controlling the sending of an ultrasound pulse and collecting reflected ultrasound signals from a sample, e.g., from inside a patient's body.

Also, the system may be configured to generate 3D spatial information from 1D data sets collected by the transducer array, or one or more sub-arrays of the transducer array.

The system may be configured to determine a position for a sub-array within the transducer array such that it is not hindered by impediments for ultrasound beams, e.g., due to ribs of a patient close to the surface or due to air bubbles between the transducer array elements of the sub-array and a sample. To collect data in order to generate, e.g., 3D spatial information, the control unit may be configured to control, for each sub-array, the location of the sub-array, as well as the focus depth and/or a steering angle for the sub-array depending on a chosen region of interest, e.g., within a patient's body.

In particular, the size and pitch of the transducer array elements may be adjusted to the wavelength, which is used for the ultrasound, i.e., the sound wave length (lambda). Herein, the pitch denotes the spacing from the center of one transducer array element to the neighboring transducer array element. The ultrasound waves, as they are emitted from (or received by) these transducer array elements interfere differently for different pitch values. For a pitch above half of the wavelength that is used for ultrasound, the waves constructively interfere at angles outside of the "main lobe", therefore causing so-called "grating lobes". Thus, a pitch value of lambda/2 or smaller may be used in order to avoid grating lobes, which may negatively impact image quality when beam steering is carried out. However, a pitch value equal to lambda may also be used, if limited beam steering capabilities and some image ghosting effects are taken into account or corrected.

For controlling a sub-array of transducer array elements in order to perform imaging, and, e.g., for measuring 3D spatial information, a positioning of the sub-array position is determined and controlled within the plurality of transducer array elements. This may be carried out by controlling a signal path between a switching array (e.g. thin-film transistor (TFT) array), which is arranged to transmit control signals to the transducer array elements and/or to transmit reading signals from the transducer array elements, and the control unit of the system, in particular a mixed signal ASIC, which may be arranged on the periphery of the sensor. The control unit, especially in combination with other elements or parts of the overall system such as (but not limited to) the switching (TFT) array, and a respective driver module for the switches (e.g. TFT's), may be configured to selectively switch rows and/or columns of transducer array elements to define an active sub-array and a sub-array location in a larger matrix of the system's transducer array.

In order to control direction and depth of a sub-array's ultrasound wave beam and controlling the ultrasound beam's profile, focusing or beam-steering or beam forming module may be used. The beam profile is controlled by applying delays on the individual transducer array elements or by applying delays on a set of combined transducer array elements which provide a common input and output, i.e. a functional unit.. This may be performed both for sending ultrasound pulses, thereby focusing ultrasound energy at a depth defined by a predetermined focal point and sending the ultrasound energy under a predetermined steering angle. Analog to the sending, the beam profile may be controlled for receiving ultrasound signals, e.g., in order to define a direction and a focal depth for receiving ultrasound signals, e.g., from a predetermined location within a sample.

The complexity of the system may be defined as a characteristic in different ways. In particular, the complexity measures the number of individually driven functional units. For example, the "complexity" of the system may refer to the total amount of channels, which are used for generating ultrasound pulses and/or for receiving ultrasound signals.

The sub-array size is chosen and configured such that a suitable signal-to-noise ratio, a specific beam profile and/or a required image quality can be achieved. For example, when a certain sub-array size SA, where SA is the product of the number of rows "n" and columns "m" (SA = n*m), is chosen, this may reduce the complexity of the system significantly.

In an embodiment, the system comprises at least two transducer arrays forming a least one transducer array set; and the control unit is configured to control the transducer array set as one single transducer array. By using a transducer array set for example several positions can be monitored (e.g. needed for lung monitoring) by means of one system. Further, a greater surface area can be monitored. Also, different viewpoints for monitoring a target, which can be an organ, fluid carrying structure or other volumetric feature in the body, e.g. the heart and/or lung and/or chest can be established.

Several transducer array's may be combined in series and/or in parallel into a single functional unit, i.e., a single transducer array set, which is driven as a single transducer array of the ultrasound system.

Different architectures may be used to address transducer array elements individually or combined in functional units. In a row-column based architecture, for example, transducer array elements that are arranged in one dimension are combined and treated as a functional unit. Such elements in one dimension may be arranged along an essentially straight line. For example, instead of controlling the single elements of a transducer array individually and/or collecting signals from all transducer array elements, all elements along one axis may be combined into a single input and/or output. For example, all elements of one row or column are combined. Connecting a transducer element to a TFT element configured as a switch, allows to selectively connect a transducer element to the row or column, so that a sub-selection can be made of the active transducers along a single row or column. The pulsers and receivers for driving respectively reading the combined transducers array elements can each be connected to either the rows or columns of the array or a combination thereof; leading to possible configurations in the read-out electronics which we designate as row/row, column/column or row/column read-out schemes.

In an embodiment, the system is configured such that the transducer array is controlled according to a row/column based architecture. This allows advantageously to reduce the data complexity greatly. The row/column architecture may be implemented by the specific transducer array architecture, or it may be implemented by how the control unit drives the transducer array, in which case the row or column configuration may be set by the control unit.

In a row/column based architecture, the transducer array elements that are arranged along the same row or column are connected together and addressed as a single functional unit. This combination of single transducer array elements reduces the complexity from N*M (when each single transducer array element is controlled individually) to N+M, wherein N and M represents the number of rows and columns present in the transducer array. Connecting a transducer element to a TFT element configured as a switch, allows to selectively connect a transducer element to the row or column, so that a subselection can be made of the active transducers along a single row or column.

Herein, each row/column unit may still consist of multiple interconnect lines forming the connections between external nodes and internal transducer array elements. Each interconnect line may herein carry a different signal, e.g., to provide a signal line, a ground line or to provide a control signal for switching the TFT transistors.

Also, an enhanced signal quality may be achieved by choosing ultrasound beam profiles with the same angle for sending ultrasound pulses and receiving ultrasound signals. To perform beam-steering for "send" and "receive" beams in the same direction, a row/row or column/column based architecture may be used. For these architectures, the "send" and "receive" channels are located along the same dimension of the transducer array, which may be configured as a 2D matrix of transducer array elements. In the row/row or column/column configuration, beam steering may be performed along one dimension. In particular, ASIC elements may be arranged along columns and/or rows of transducer array elements to allow beam-steering in two orthogonal directions. However, for each event of sending and receiving, e.g., to collect image data, only one of the two orthogonal directions may be accessed.

In particular, the elements of one row or column may be combined in different ways. For example, data of these elements as sensors may be virtually combined into one channel by a suitable control by the control unit, or the row/column architecture may be implemented by the design of the transducer array itself, e.g., by performing an integration step directly on the transducer array element level. A row/column configuration may also be implemented by a suitable chip design.

The sub-arrays are electronically defined by a combination of control signals on the TFT array (determining which transducer element are active) and the routing of the pulsing channels and readout channels to the TFT arrays or columns. For example, without limiting the invention to these features, the TFT driver, which may be comprised by the control unit and/or partly by circuitry on the TFT layer, can select the active rows, while the control layer connects the read and write channels to the columns. In particular, the intersection of both will form the sub-array. In another example, the TFT driver selects the active columns, while the control unit connects the write channel on the rows and the read channels of the columns or vice versa. Also, the elements of several adjacent rows or columns may be combined in the same manner.

In an embodiment, the system is configured such that a sub-array size is provided, sub-arrays of the sub-array size are defined and the transducer array is controlled based on the individual sub-arrays. The sub-arrays can therefore advantageously be defined very precisely and adjusted to different conditions.

The value of sub-array size may be determined or given in different ways, for example in a dynamic determination step, in which an optimum sub-array size is determined depending on parameters like free computing capacity, signal-to-noise ratio or accuracy values, geometric properties of an object or of the transducer array, or calibration values, which may be determined or measured in a calibration step. In particular, the sub-array size is determined beforehand through system studies. Considerations may be, for example, the signal to noise ratio of the return signal and the ultrasound beam profile, including parameters such as beam spreading and focus beam width. Another parameter may be the sub-array size, if this is chosen too large, the sub-array would not fit in between the ribs of a patient, on whom the system is applied.

For example, a sub-array size of at least 4x4 (rows x columns), 8x8, 10x10, 12x12, 14x14, 16 rows by 16 columns may be provided. The chosen value may depend on the wavelength that is used for the ultrasound. For an array, in which the transducer array elements are spaced apart about a full wavelength, combining a larger number of transducer elements may create problems, when measuring through ribs, which have a certain distance to each other.

In particular, the control unit may provide beam characteristics for each sub-array. Thus, the elements of this sub-array are used to generate ultrasound pulses in one specific direction and with defined pulse properties, respectively. On the other hand, the beam characteristics define ultrasound signal detection properties of the system, in particular the direction and depth where ultrasound signals are detected.

In an embodiment, the transducer elements of each sub-array are configured to provide an analog ultrasound sensor signal and the ultrasound sensor signals of the transducer elements of each sub-array are provided to one common analog-digital converter (ADC). In particular, the individual signals of the transducer array elements of one sub-array are combined first, before they are provided to the ADC. This leads advantageously to a reduced complexity.

For example, there may be one ADC per row or per column in a row/column architecture. The ADC may be comprised by the data processing unit or implemented as separate units. By providing the detected signals of a sub-array of transducer array elements to one ADC, only one digital signal is produced and the amount of data that is acquired is reduced.

In an embodiment, a beam forming architecture is implemented by a hardware beam forming module. In other words, the beam forming on a sub-array level is performed not or not only via signals from the control unit, but by a hard-wired chip design.

In particular, delays are defined for the inputs and/or outputs within one sub-array, such that a defined beam form is reached for ultrasound signals generated or detected by the transducer elements, connected to these inputs/outputs. In the analog domain, sensor signals for all sub-array outputs are delayed and summed into a single output channel. Signals from this common output channel may be provided to one ADC and converted to a digital signal for further data treatment, filtering, imaging and monitoring of measurements.

The number of analog-digital converters that are needed by the system is reduced from one per receive channel to one per sub-array, thus also reducing the amount of data that is generated. Consequently, significant benefits in power consumption and data rate may be reached.

Additionally or alternatively, a software or programming implementation of sub-array beam forming may be used in the system.

In an embodiment, the system is configured to filter data received from the transducer array and to obtain filtered data with a reduced spatial and/or temporal resolution. This leads advantageously to a reduced amount of data to be treated. The filtering may be carried out by the data processing module and/or by other modules and units.

Different filter circuits and/or algorithms may be applied to reduce the amount of data that needs to be provided by the system. For example, a sampling rate may be adjusted by hardware and/or software measures such that measurements are only carried out at the time points that are actually needed. Also, the spatial resolution of measurements and scans may be adjusted to suitable values, e.g., depending on data processing capabilities, power consumption requirements or quality requirements for a subsequent data.

Also, the invention relates to a method for operating an ultrasound system. Herein, a plurality of sub-arrays is defined within a transducer array. The individual sub-arrays are controlled as functional units for sending ultrasound pulses and/or receiving ultrasound signals.

In particular, the method serves the purpose of operating the ultrasound system. Thus, it has the same advantages as the ultrasound system of the invention.

Further details and advantages of the present invention shall now be disclosed in the connection with the drawings.

It is shown in
- **Fig. 1**: an embodiment of the ultrasound system;
- **Fig. 2A-D**: detailed views of the transducer array of the embodiment of the ultrasound system;
- **Fig. 3**: the embodiment of the ultrasound system as shown in Fig. 1; and
- **Fig. 4**: an embodiment of a method to operate the ultrasound system; and

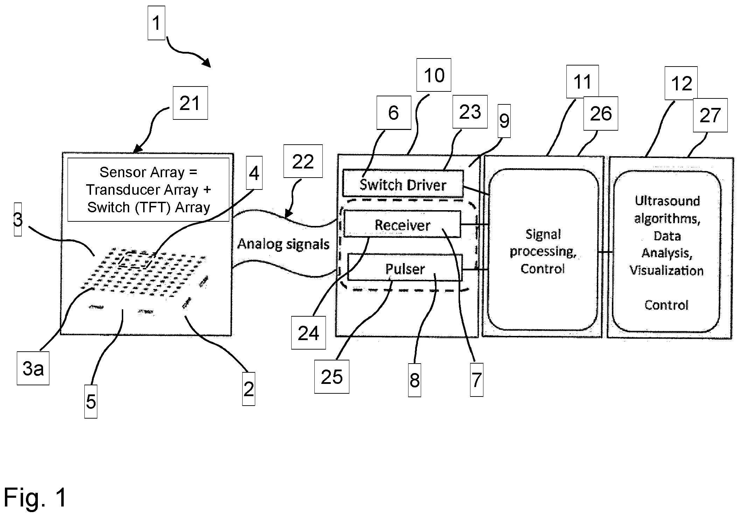

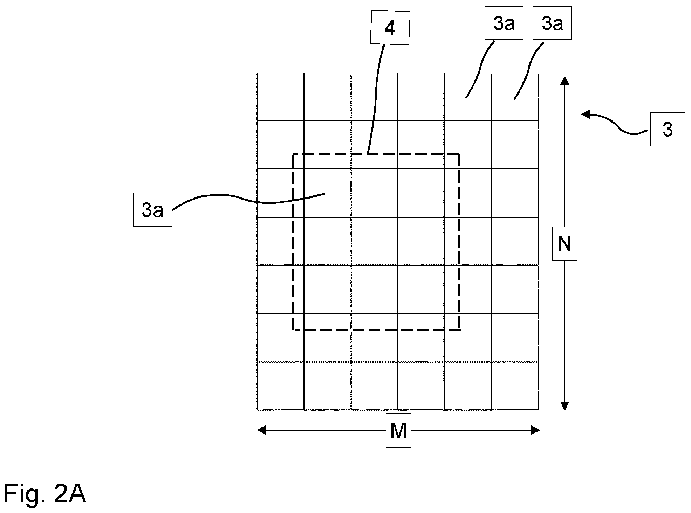

Turning to **Fig. 1** and **Fig. 2A** to **Fig. 2C****,** an embodiment of the ultrasound system for intercostal imaging of organs, here for cardiac monitoring is described.

It can be attached to the skin of the patient and then an intercostal imaging of organs, here cardiac monitoring, can be performed.

Also, any other intercostal imaging of organs can be performed.

**Fig. 1** shows a schematical overview and explaining the functional blocks of the ultrasound system 1.

The ultrasound system 1 according to the embodiment is configured as a wearable ultrasound system 1, in particular for long-term monitoring of a patient's cardiac activity.

In particular, the ultrasound system can be in general an ultrasound system as disclosed by WO 2019/162485 A1. The below additional features can be further realized in this already disclosed ultrasound system of WO 2019/162485 A1.

The ultrasound sensor and support electronics can be integrated in a flexible body worn format, as it is shown in this embodiment.

In the embodiment as shown in **Fig. 1****,** the ultrasound system is (at least partially) carried in a patch 2.

The ultrasound system 1 comprises a transducer array 3, which is configured as a piezoelectric micromachined ultrasonic transducer (pMUT) array 3 in the present embodiment.

The transducer array 3 comprises a plurality of transducer array elements 3a.

In the present embodiment pMUT elements 3a.

A subset of the transducer array elements 3a forms a sub-array 4.

In particular, each transducer array element 3a can consist of one or several sub-elements 3b, in particular pMUT sub-elements 3b or components, which are driven as a single larger transducer array "element" 3a.

In other embodiments, capacitive micro-machined ultrasonic transducer (cMUT) components can be used.

Other configurations with different numbers of transducer sub-elements 3b or a different arrangement of the sub-elements 3b are possible as well.

Furthermore, there is a switch array or switch module 5, which can more specific be embodied as a TFT array.

A further part of the ultrasound system 1 is the Analog Front-End 10 with the Switch Driver 6, the Receiver 7 and the Pulser 8.

The Switch Driver 6 can be embodied as a TFT driver module.

The Receiver 7 can be embodied as analog-digital converter (ADC).

The Pulser 8 can be a pulser module.

There can be a digital application-specific integrated circuit (ASIC) 9.

Further, there is an interface module 11 and an external unit 12/back-end unit 12.

A transducer array 21, which comprises a sensor array (e.g., like the transducer array 3 described above) and a switch/TFT array, is coupled with a unit implementing a switch driver 23, a receiver 24 and a pulser unit 25.

These units are configured to communicate via analog signals 22 with the transducer array 21.

In the embodiment, they are implemented in application-specific integrated circuits. Such ASICs can be located on the periphery of the sensor or on the Analog Front-End 10.

Also, they are used to receive and treat the signals, and communicate with a signal processing/control unit 26, which is implemented in an embedded compute system, e.g. on the Analog Front-End 10 with CPU, FPGA with memory or other units, these control and signal processing algorithms might also be implemented partly or completely in an ASIC.

Further processing of provided data is performed by a control module 27, incorporating for example ultrasound image reconstruction algorithms, data analysis, visualization and control algorithms.

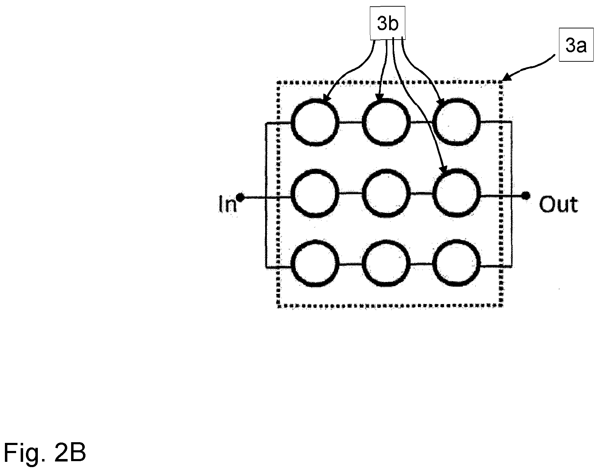

In **Fig. 2B****,** one example of a possible transducer array element design is shown: Nine sub-elements 3a (pMUT, cMUT or other elements) are configured to form one transducer array element 3a. To this end, they are arranged in three rows and three columns. Therein, the sub-elements 3 of one row are connected in series, and the rows of sub-elements 3a in parallel. Thus, three parallel branches of each three sub-elements 3b connected in series are connected. Other configurations are possible as well. In total, the transducer array element 3a has one "In" and one "Out" line. Herein, the sub-elements 3b are not controlled individually, but the transducer array element 3a is controlled as a whole.

In the present embodiment, the transducer array 3 comprises 150 * 150 pMUT elements 3a, which are arranged in a 2D matrix with perpendicular rows and columns. The array size may differ in different embodiments.

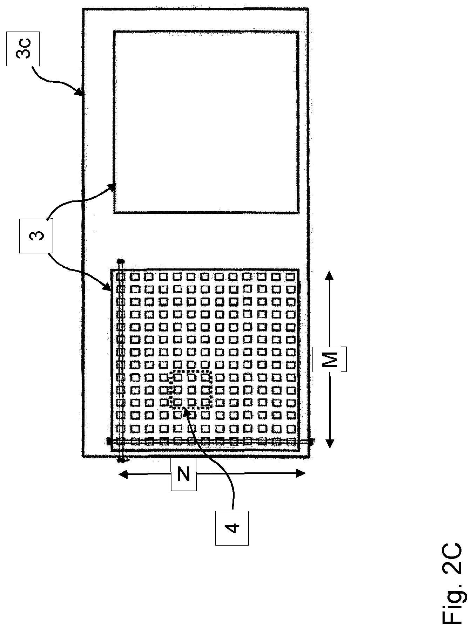

Furthermore, **Fig. 2C** shows another embodiment, wherein two or more transducer arrays 3 can form a transducer array set 3c, which is then controlled as one functional unit, i.e. "virtual" single transducer array.

In the embodiment of **Fig. 2C****,** a first and a second transducer array 3 are comprised by one transducer array set 3c. In this embodiment, the transducer array set 3c is controlled as "virtually" one single transducer array 3.

Also, **Fig. 2C** shows that the transducer array 3 comprises a plurality of transducer array elements 3a, which are arranged in a matrix with a number of N rows and M columns.

Furthermore, **Fig. 2C** shows that a subset of the transducer array elements 3a make up a sub-array 4, which is herein controlled as one unit. The sub-array 4 has a number of n rows and m columns.

**Fig. 2C** also shows that elements 3a along a row and/or a column of the transducer array 3 are interconnected with one or more interconnecting lines, a so called row/column based architecture. The elements 3a connect in parallel to the interconnects, with a switch, in particular a TFT switch, determining if an element 3a is connected or not and to which signal path it connects. Thus, rows and/or columns of the transducer array 3 can be controlled together. This allows simplifying the system by switching rows and/or columns of elements 3a together.

The transducer array 3 of the embodiment is flexible. Here, the transducer array elements 3a are arranged on a flexible substrate, e.g., comprising a flexible polymer material.

Furthermore, the ultrasound system 1 comprises a switch array which can be embodied as a thin-film transistor (TFT) array 5, comprising a plurality of TFT elements.

The switch array 5 is arranged such that its switch elements (or TFT elements when embodied as a TFT array) control corresponding pMUT elements 3a. In particular, each TFT element is assigned to one pMUT element 3a.

In the present embodiment, a TFT array 5 with 150 * 150 TFT elements is used, which are arranged in a 2D matrix with perpendicular rows and columns. The array size depends in particular on the size of the respective pMUT array 3, and it may differ in different embodiments.

Similar to the transducer array 3, the TFT array 5 of the embodiment is also flexible and/or stretchable. Here, the TFT array elements are arranged on a flexible substrate, e.g., comprising a flexible polymer material.

In particular, the TFT array 5 is arranged on the same substrate as the transducer array 3.

In particular, the integration may be monolithic or heterogeneous. In the present exemplary embodiment, the sensor layer and TFT layer may be fabricated separately and joined in a wafer-to-wafer bonding process, followed by the processing of an interconnect module.

In particular, "Heterogeneous Integration (HI)" may refer to the assembly and packaging of multiple separately manufactured components onto a single chip in order to improve functionality and enhance operating characteristics. In the present embodiment, one single array is created with a single functionality (sensing), implying a monolithic device.

In particular, attention is drawn to **Fig. 2C****,** wherein an example of a row/column architecture is demonstrated.

In the embodiment, both the pMUT array 3 and the TFT array 5 are integrated into an adhesive patch 2.

In the embodiment, the adhesive patch 2 has a size of about 10x10 cm. The size may vary in different embodiments.

The adhesive patch 2 comprises a material that is suitable for the purpose of attaching the ultrasound system 1 and in particular the transducer array 3 to a patient's skin. Therefore, biocompatible and breathable materials are used. The adhesive layer on the patch is used to attach the patch to the body and to make sure that the position of the transducer array 3 is essentially constant with respect to the patient's skin.

In another embodiment, an "acoustic matching layer" is provided in between the skin and the transducer array 3.

The system 1 also comprises a functional block for the analog control of the electronics, a so called Analog Front-End.

The Analog Front-End 10 is coupled with the transducer array 3 and the TFT array 5 for providing options such as control and read-out for the transducer array 3 and/or the TFT array 5. To this end, both data and electrical power may be transferred over the coupling between the Analog Front-End 10 and the patch 2 with transducer array 3 and TFT array 5.

According to the embodiment, the Analog Front-End can be implemented in an application specific IC (ASIC) or as circuits on the TFT layer or as discrete components or a combination thereof. All the physical components of which the analog front-end is comprised can be either integrated directly on the TFT as integrated components, on the periphery of the TFT as IC's or on a PCB (flex or rigid) physically attached to the sensor 2 through physical interconnects.

In different embodiments, the application specific IC's may be either integrated on the periphery of the TFT layer or on the PCB.

Here, a TFT driver module 6 is provided on the Analog Front End 10, which is configured to provide a low-level interface to control and manage the TFT array 5 and its individual TFT elements, respectively. In an embodiment, a gate driver is integrated with the ASIC on the periphery of the TFT layer. In another embodiment the driver circuitry could be designed partly or completely in the TFT layer.

In particular, the TFT driver module 6 of the present embodiment can be integrated together with send and receive modules for controlling the generation of ultrasound pulses and detecting ultrasound signals, respectively. All these functional modules form part of the analog front-end.

Also, a receiver 7 is provided as part of the analog-front end 10, which is configured to convert an analog input signal to a digital output signal.

Also, a pulser module 8 is provided as part of the analog front end 10, which is configured to control the transducer array 3 such that ultrasound pulses are generated. In particular, a beam-forming is performed here. Delay values may be set individually for each pulser unit of the system 1 that generates an ultrasound pulse. The delays on the different channels, in particular each pMUT element 3a, of the pulser unit will determine the focusing and the beam profile.

Also, a digital middle-end is provided , which is configured to perform part of the control operations for the ultrasound system 1, and specifically to control the activity of the transducer array 3 and the TFT array 5 for generating ultrasound pulses and detecting ultrasound signals. The digital middle-end is also configured to perform signal processing on the signals received from the analog front-end.

As part of the digital middle end, microprocessors may be provided and/or other units for performing operations for controlling the ultrasound system 1 and its electronic components.

Thus, the ultrasound system 1 of the embodiment provides an easily customized ultrasound sensor and monitoring device.

The interface module provides the possibility to build up a connection based on a wire connection and/or a wireless connection. In the present embodiment, examples for connection methods include USB and Ethernet as well as via Bluetooth and WiFi networks.

The interface module is here configured to allow for data communication and/or power connections with external units.

In the embodiment, the PCB with its components and the TFT array 5 constitute an integrated control unit and data processing unit. PCB and TFT array 5, and the transducer array 3, may in different embodiments, be configured as units that are formed separately or they may be incorporated in a common unit.

Turning to **Fig. 2A****,** a detailed view of the transducer array 3 of the embodiment of the ultrasound system 1 is described.

The transducer array 3 has a matrix of pMUT elements 3a, which are only schematically shown in a small number.

The system 1 is configured such that a sub-array 4 of pMUT elements 3a may be defined. The elements 3a of a sub-array 4 are treated as one functional unit for generating ultrasound pulses and/or detecting ultrasound signals.

In the embodiment, an ASIC performs steps for defining sub-arrays 4 within the transducer array 3. The definition of sub-arrays 4 may be carried out in different ways, e.g., by way of the chip design or by other units controlling the transducer array 3.

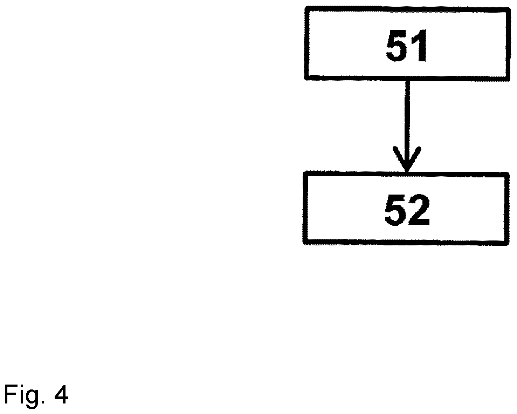

In the present embodiment, the transducer array 3 has between N=75-200 rows and M=75-200 columns, which are ordered in a two-dimensional matrix. On the other hand, a sub-array size value of 4 to 32 rows by 4 to 32 columns is defined. Thus, groups of 162 elements 3a are grouped into one sub-array 4. This leads to a considerable reduction in system complexity and data volume, respectively. This aspect is further described below with reference to **Fig. 4****.**

Herein, the interrelated elements 3a are chose such that they are neighboring to each other.

In further embodiments, this definition of sub-arrays 4 may be configured in different ways, such as, in a configuration step, by inputting a predetermined sub-array size value, which defines the number of pMUT elements 3a to be included in a sub-array 4, and/or by inputting a location of a sub-array 4 within the pMUT array 3. Also, the form of a sub-array 4, i.e., its dimensions size, may be determined by an input value.

In one embodiment, the sub-array size may be defined during the system development. In the products itself, the sub-array size can be fixed, without an option to dynamically reconfigure this value; in this case, a fixed size would be used.

Said values to control how sub-arrays 4 are defined may also be determined by the digital Middle End and/or digital back-end, part of this functionality might be implemented in an ASIC or another control element of the ultrasound system 1, or by an input over the interface module, in particular depending on an algorithm. Such an algorithm for determining parameters for defining the sub-arrays 4 may use input values such as data/image quality requirements, a signal-to-noise ratio, information about factors interfering with the operation of the system, user input and configuration information, and/or other parameters.

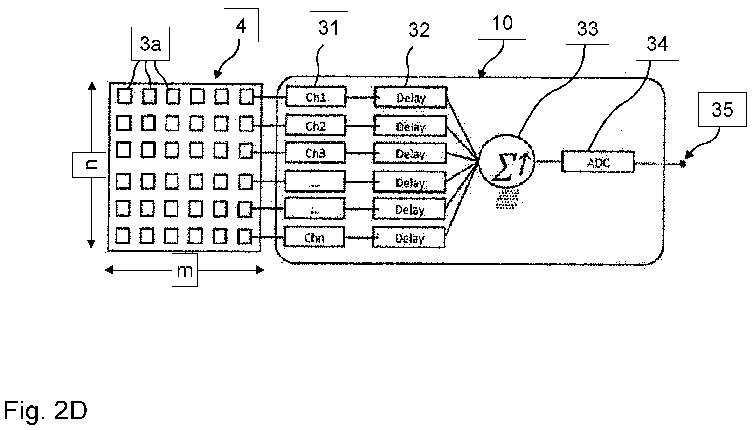

Turning to **Fig. 2D****,** an embodiment for implementing a row/column based micro beam-former is schematically described.

A sub-array 4 has a number of n row and m columns, and is connected to a micro beam-former 30. In this specific case, the n rows of transducer array elements 3a are combined into one output channel 31 each. These output channels are named "Ch1" to "Chn".

For the transducer array element 3a of the sub-array 4, the micro beam-former 30 combines the n analog input channels 31 from the sub-array 4 into one single digital output channel 35.

To this end, a delay operation is performed by delay units 32 and a sum unit 32 is subsequently used to coherently add the n analog inputs together. An analog-digital converter 34 is used to convert the resulting analog signal to a digital signal, which is output through a digital output channel 35.

In further embodiments, each channel 31 can do some analog processing of the input signals before its signal is combined in the analog beam-former 30.

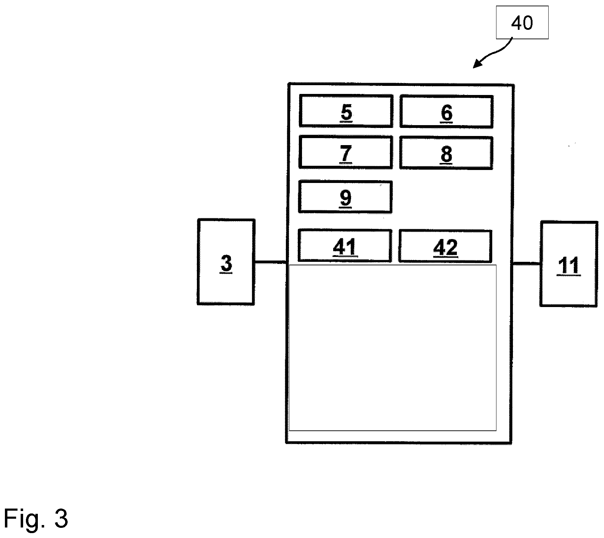

In **Fig. 3****,** a schematic view of another embodiment is shown. The shown components may also be present in the embodiments described above; similar or equivalent elements have the same reference numerals and are not described again in detail.

Some or all of the elements and modules shown in **Fig. 3** may be implemented in different units, such as separately formed devices, or they may be implemented by way of program modules within the same computational unit and/or a digital memory device.

The combination of elements and modules of this embodiment is to be understood as exemplary, and as comprising a large number of optional features, which are not necessarily linked to each other or to be combined in one embodiment of the invention.

In **Fig. 3****,** the transducer array 3 is shown, which is linked to a control unit 40 (controlling the transducer array 3), which may be configured at least partially on a PCB 10.

The connection between the transducer array 3 and the control unit 40 is a direct and cable-bound connection. However, in an alternative embodiment, an indirect and/or wireless connection is possible.

For instance, the control unit 40 can be attached to the patch 2 (possible for wireless patches and wired patches). Further, there can be an additional control unit in a separate computer, in particular for the wired version.

The transducer array 3, configured for providing ultrasound waves and/or receiving ultrasound signals, comprises a plurality of transducer array elements 3a.

The control unit 40 comprises the TFT array 5, which was already described above.

The control unit 40 further comprises a dedicated data processing unit 41, which may in different embodiments comprise some of the other modules (as specified below with reference numerals 41 to 49) or which may in different embodiments be implemented by several individual modules. The data processing unit 41 receives and processes data from the transducer array 3.

Moreover, the control unit 40 comprises a reference module 45.

Also, the control unit 40 comprises a complexity reduction module 42.

Combining of groups of transducer elements 3a into a sub-array 4 can alternatively be done by the complexity reduction module 42.

Also, the complexity reduction module 42 may be implemented as a software module, comprising instructions for computational operations that implement the function of complexity reduction. Such a software module may in particular be implemented together with the control unit 40.

Specifically, the complexity reduction module 42 may be comprised, at least partially, by the control unit 40 and/or by the data processing unit 41.

As shown in **Fig. 3****,** the ultrasound system 1, in particular for cardiac monitoring, comprises a transducer array 3 with a plurality of transducer array elements 3a, a control unit 40, which is coupled to the transducer array 3, for controlling the transducer array 3, and a data processing unit 41 for receiving data from the transducer array 3 and processing the received data. Therein, the ultrasound system 1 further comprises a complexity reduction module 42, which is configured to define at least one sub-array 4 of transducer array elements 3a and to control the at least one sub-array 4 as a functional unit for sending at least one ultrasound pulse and/or receiving at least one ultrasound signal.

In one embodiment, the ultrasound system 1, in particular for cardiac monitoring, comprises a transducer array 3 with a plurality of transducer array elements 3a for providing ultrasound waves, a control unit 40, which is coupled to the transducer array 3, for controlling the transducer array 3, and a data processing unit 41 for receiving data from the transducer array 3 and processing the received data.

In **Fig. 4****,** a method for operating the ultrasound system 1 is described. The following description is based on the embodiment of the ultrasound system 1 described above with reference to, e.g., **Fig. 3****,** but other embodiments of the ultrasound system 1 may be suited as well.

In this context, "complexity reduction" denotes a reduction of the amount of read and/or write channels needed to address the sensors, in particular the transducer array elements 3a or a sub-array 4.

This can be reached by
a) combining sub-elements 3b into a single functional element 3a (cf. **Fig. 2B****);**
b) connecting elements 3a together in a row/column architecture, leading to a complexity reduction from N*M to (N+M) (cf. **Fig. 2C****);**
c) driving a sub-array 4 instead of the full transducer array 3 at once, leading to a complexity reduction from (N+M) to (n+m); and/or
d) using a micro beam-former to combine the n (or m) analog input channels 31 of a sub-array 4 into a single digital input channel 35 (cf. **Fig. 2D****).**

In a step 51, a plurality of sub-arrays 4 is defined within the transducer array 3. In a subsequent step 52, the individual sub-arrays 4 are controlled as functional units for sending ultrasound pulses and/or receiving ultrasound signals.

In the system of the embodiment, the steps 51, 52 are performed by the complexity reduction module 42, which is comprised by the control unit 40 of the ultrasound system 1. Herein, the complexity reduction module 42 is configured to control other units and modules, especially the TFT driver module 6, the receiver module 7, and/or the pulser module 8, depending on whether a send or a receive cycle is active.

The steps 51, 52 may be performed in a cyclic manner, i.e., they may be repeated for example at given intervals or triggered by predefined events and/or control signals.

In one embodiment, the system is configured such that the sub-array 4 is defined dynamically and may change its position.

In the present embodiment, the sub-arrays 4 are defined such that there is no overlap between the transducer array elements 3a of separate sub-arrays 4, i.e., no transducer array elements 3a are "shared" among different sub-arrays 4.

In another embodiment, a transducers array set 3c comprises at least two transducer arrays 3 and the control unit 40 is configured to control the transducer arrays 3 as one functional unit.

Also, in an embodiment, the transducer array 3 may be controlled according to a row/column based architecture.

Furthermore, a sub-array size may be predetermined or provided or determined by an algorithm, and sub-arrays 4 of the sub-array size are defined based on this value. The transducer array 3 is then operated based on these individual sub-arrays 4.

Also, the transducer elements 3a are controlled by the control unit 40, in particular via the TFT array 5. The transducer array elements 3a of each sub-array 4 detect an analog ultrasound sensor signal, and produce an analog signal. This analog signal is provided to the receiver module 7.

In particular, analog signals from the transducer array elements 3b of one sub-array 4 are processed by one common receiver module of the system 1. In further embodiments, different receiver modules 7 may be provided, e.g., in order to speed up data acquisition and processing.

Furthermore, a micro beam-forming architecture can be implemented by a hardware beam-forming module 43, such as a specialized ASIC element and/or another element of the control unit of the system 1.

Also, data may be filtered after it has been received from the transducer array 3. This may be carried out by a filter module 44, which may act on analog and/or digital signals. Depending on which filter is applied, filtered data is obtained with a reduced spatial and/or temporal resolution.

In another embodiment, a filter can be configured by adjusting filter properties. Thus, filters may be activated and deactivated, the impact of a filter on the data may be adjusted and/or a resolution of the filtered data may be adapted, e.g., to the needs for monitoring specific organs and functions, imaging purposes, or device-specific properties.

### Reference numerals

- 1: Ultrasound system
- 2: Adhesive patch
- 3: transducer array; pMUT array
- 3a: transducer array element; pMUT element; transducer array unit
- 3b: sub-element; pMUT sub-element
- 3c: transducer array set
- 4: sub-array
- 5: thin-film transistor (TFT) array
- 6: TFT driver module
- 7: analog-digital converter (ADC)
- 8: pulser module
- 9: digital application-specific integrated circuit (ASIC)
- 10: Analog Front-End
- 11: interface module
- 12: external unit; back-end unit
- 21: transducer array
- 22: analog signals
- 23: switch driver unit
- 24: receiver unit
- 25: pulser unit
- 26: signal processing; control unit
- 27: control module
- 30: micro beam-former
- 31: analog output channel
- 32: delay unit
- 33: sum unit
- 34: analog-digital converter (ADC)
- 35: digital output channel
- 40: control unit
- 41: data processing unit
- 42: complexity reduction module
- 51: step
- 52: step
- 71: step
- 72: step
- 73: step
- 74: step
- 75: step
- 76: step
- 77: step
- 81: step
- 82: step
- 83: step
- 84: step
- 91: step
- 92: step
- 93: step
- N: number of rows
- M: number of columns

## Claims

1. Ultrasound system (1) for intercostal imaging of organs, in particular for cardiac monitoring, comprising:
- a transducer array (3) with a plurality of transducer array elements (3a);
- a control unit (40), which is coupled to the transducer array (3), for controlling the transducer array (3); and
- a data processing unit (41) for receiving data from the transducer array (3) and processing the received data;
wherein the ultrasound system (1) further comprises a complexity reduction module (42), which is configured to define at least one sub-array (4) of transducer array elements (3a) and to control the at least one sub-array (4) as a functional unit for sending at least one ultrasound pulse and/or receiving at least one ultrasound signal,
wherein further the complexity reduction module (42) is configured to combine several transducer array elements (3a) into a functional unit, which then can be handled as if it were one single transducer array element (3a), and wherein the complexity reduction module (42) is configured to read and/or drive and/or control such functional units.

2. Ultrasound system (1) according to claim 1,
**characterized in that**
the transducer array (3) is flexible.

3. Ultrasound system (1) according to one of the preceding claims,
**characterized in that**
the transducer array (3) is integrated into a patch (2) for fixing the transducer array (3) to a patient's body, especially wherein the patch (2) is an adhesive patch (2).

4. Ultrasound system (1) according to one of the preceding claims,
**characterized in that**
the coupling between the transducer array (3) and the control unit (40) comprises a wire connection and/or a wireless interface.

5. Ultrasound system (1) according to one of the preceding claims,
**characterized in that**
the system further comprises a beam former module; wherein the beam former module is configured to apply a time delay to at least one transducer array element or a plurality of transducer array elements.

6. Ultrasound system (1) according to one of the preceding claims,
**characterized in that**
the system further comprises a power source unit for providing energy to the transducer array (3) and/or the control unit (40).

7. Ultrasound system (1) according to one of the preceding claims,
**characterized in that**
the complexity reduction module (42) is configured to define the plurality of sub-arrays (4) such that there is no overlap between the transducer array elements (3) of separate sub-arrays (4).

8. Ultrasound system (1) according to one of the preceding claims,
**characterized in that**
the system comprises at least two transducer arrays (3), forming a least one transducer array set; and the control unit (40) is configured to control the transducer array set as one single transducer array (3).

9. Ultrasound system (1) according to one of the preceding claims,
**characterized in that**
the ultrasound system (1) is configured such that the transducer array (3) is controlled according to a row/column based architecture.

10. Ultrasound system (1) according to one of the preceding claims,
**characterized in that**
the ultrasound system (1) is configured such that a sub-array size is provided; sub-arrays (4) of the sub-array size are defined; and the transducer array (3) is controlled based on the individual sub-arrays (4).

11. Ultrasound system (1) according to one of the preceding claims,
**characterized in that**
the transducer array elements (3a) of each sub-array (4) are configured to provide an analog ultrasound sensor signal; and the ultrasound sensor signals of the transducer array elements (3a) of each sub-array (4) are provided to one common analog-digital converter (7, 34).

12. Ultrasound system (1) according to one of the preceding claims,
**characterized in that**
a beamforming architecture is implemented by a hardware beamforming module.

13. Ultrasound system (1) according to one of the preceding claims,
**characterized in that**
the system is configured to filter data received from the transducer array and to obtain filtered data with a reduced spatial and/or temporal resolution.

14. A method for operating an ultrasound system (1), especially an ultrasound system according to one of the preceding claims, comprising the following steps:
- defining a plurality of sub-arrays (4) within a transducer array (3); and
- controlling the individual sub-arrays (4) as functional units for sending ultrasound pulses and/or receiving ultrasound signals.
